# EUROPÄISCHE PATENTANMELDUNG

(11) **EP 1 995 587 A1**
(43) Veröffentlichungstag der Anmeldung: **26.11.2008**
(21) Anmeldenummer: 08161356.4
(22) Anmeldetag: 09.03.2007
(51) Int. Cl.: G01N 21/64, G01N 1/31, G01N 33/542, G01N 33/58

(54) **Vorrichtung und Verfahren zur Erkennung und Identifizierung von Zielstrukturen**

(30) Priorität: 13.03.2006 DE 102006011467
(62) Teilanmeldung aus: 07711875.0
(71) Anmelder: Schubert, Adrian, 39108 Magdeburg (DE)
(72) Erfinder: Schubert, Adrian, 39108 Magdeburg (DE)
(74) Vertreter: Charrier, Rapp & Liebau

(57) **Zusammenfassung**

Die vorliegende Erfindung betrifft eine automatisierte Vorrichtung und ein Verfahren zur Bestimmung und Messung einer beliebigen Anzahl Xn (n = 1,2,3...N) von Zielstrukturen bestehend aus Molekülklassen, Molekülgruppen und Molekülteilen auf einem flüssigen oder festen Objekt bzw. in einem flüssigen oder festen Objekt, wobei die Vorrichtung eine Kombination aus einem Pipettiersystem, einem 3D-Handhabesystem und einem optischen Messsystem umfasst, wobei das Pipettiersystem, das 3D-Handhabesystem und das optische Messsystem durch einen Computer gesteuert und kontrolliert werden und das Pipettiersystem eine automatische Pipettierung für die Aufnahme und Abgabe von Flüssigkeiten und eine angemessene Wiederholpositioniergenauigkeit, bevorzugt von mindestens +/- 0,1 mm, aufweist, dadurch gekennzeichnet, dass das Pipettiersystem eine manuell oder automatisch vor und nach dem Einsatz des Vorrichtung aufsetzbare und abnehmbare oder fest installierte Pipettenspitze aufweist, derart, dass während des Einsatzes der Vorrichtung kein Pipettenwechsel erfolgt.

## Beschreibung

Nach der Entschlüsselung des humanen Genoms besteht die nächste Herausforderung darin, die Protein-Netzwerke einer Zelle zu entschlüsseln, und damit die exakte zelluläre (vs. molekulare) Funktion jedes einzelnen Proteins als Funktion des Kontextes und der Interaktion mit anderen Proteinen zu verstehen. Dazu müssen völlig neue Werkzeuge entwickelt werden. Im Zentrum der zu lösenden technischen Probleme steht die Notwendigkeit Vorrichtungen und Verfahren zu entwickeln, die es ermöglichen eine praktisch beliebige Anzahl verschiedener Proteine oder andere Zielstrukturen in ein und derselben Zelle in nur einem einzigen Experiment zu lokalisieren. Dadurch erst werden die Regeln der räumlichen Assoziationen der Moleküle, Molekülgruppen und insbesondere der Proteine bezogen auf Strukturen erkennbar, so dass in Kombination mit Experimenten (z.B. "Protein Knock Out") oder durch Vergleich verschiedener zellulärer Zustände, wie z.B. in Krankheit und Gesundheit, die exakten zellulären Funktionen dieser Assoziationen entschlüsselt werden können.

Das Problem der Kartierung einer sehr großen Zahl von Molekülen, Molekülgruppen oder insbesondere Proteinen in ein und derselben Zelle ist durch die Entwicklung eines Verfahrens und einer Vorrichtung, wie sie in der EP 0 810 428 B1 beschrieben sind ("Multi-Epitop-Ligand Kartographie" (MELK)) vom Grundsatz her gelöst.

Die Anwendung dieses Verfahrens hat darüber hinaus zu wichtigen neuen Erkenntnissen über die funktionelle Architektur von zellulären Protein-Netzwerken sowie zur Identifikation kombinatorischer Proteinmuster bei Krankheiten geführt (vgl. US 6,638,506, US 6,649,165, US 6,638,515 sowie Schubert W.: "Topological Proteomics, Toponomics, MELK Technology", In: Adv. Biochem. Eng. Biotechnol. 83: 189 - 209 (2003)).

In der Praxis der Anwendung dieses Verfahrens bestehen allerdings noch zahlreiche technische Limitierungen, die den Eintritt dieser Technologie in eine Routineanwendung im Labor, sei es in einem Forschungslabor oder einem Diagnoselabor, erschweren. Dazu zählen folgende Einschränkungen:
1. Durch den in EP 0 810 428 B1 beschriebenen Wechsel von Pipettenspitzen wird relativ viel Zeit auf einen technischen Vorgang verwendet, so dass Zeit für die Markierung von Molekülen, Molekülgruppen oder Proteinen verloren geht. Außerdem ist der Vorgang fortgesetzter Pipettenspitzenwechsel anfällig bezüglich möglicher Ausfälle (Störungen des Pipettenspitzenabwurfs, Störungen der Pipettenspitzenaufnahme etc; nicht korrekt sitzende Pipettenspitze an der Saugeinrichtung des Pipettenspitzen-Aufnahmegerätes mit der Folge des Ansaugens von Luft etc).
2. Die Vorgänge der Markierung von Proteinen und Visualisierung des Markierungsresultates, so wie sie in EP 0 810 428 B1 beschrieben, erfordern pro Einzelmarkierung - bevor ein weiteres Protein markiert werden kann - relativ viel Zeit, so dass derzeit in der Praxis nicht mehr als zwei Proteine pro Zell- oder Gewebeprobe pro Stunde markiert werden können. Diese Tatsachen stehen einer Übersetzung dieser Technologie in einen den Hochdurchsatzverfahren ähnlichen Durchsatzbereich derzeit noch entgegen.
3. Die Bestimmung von Schwellenwerten für die einzelnen markierten Moleküle, Molekülgruppen oder Proteine, ist nicht automatisiert, da es an objektiven Kriterien für eine Diskriminierung von Hintergrund und Vordergrund fehlt. Daraus resultiert insbesondere im unteren Grauwertbereich eines digitalisierten Molekül-, Molekülgruppen- oder Proteinsignals eine große Unsicherheit bezüglich der Entscheidung, ob ein Molekül, eine Molekülgruppe oder ein Protein vorhanden ist oder nicht. Im Ergebnis können auf diese Weise noch keine automatisierten Datensätze mit hinreichenden, biologisch basierten Kriterien der Kombinatorik der Moleküle, Molekülgruppen oder insbesondere der Proteine erstellt werden. Diese Tatsache steht vorerst einer klinischen Anwendung, die eine hohe Robustheit und Automatisierung erfordert, entgegen.

Die im Folgenden beschriebenen erfindungsgemäßen Vorrichtungen und Verfahren stellen eine Überwindung der genannten Einschränkungen dar.

1. Automatisiertes Verfahren gemäß EP 0 810 428 B1 zur Durchführung der oben als Stand der Technik angegebenen repetitiven Inkubations-Imaging-Bleaching Zyklen (RIIBZ), mit dem Unterschied, dass in dem automatisierten Prozess kein Pipettenwechsel mehr stattfindet: also alle technischen Parameter in dem Kapitel 0027 des o.g. Patentes sollen beibehalten werden, lediglich Punkt 2 wird dahingehend geändert, dass ein Pipettier-Roboter (3D-Handhabesystem oder ähnlicher Roboter) mit ein- und derselben Pipettenspitze arbeitet, also kein automatisierter Pipettenwechsel mehr stattfindet. Zwischen den einzelnen RIIBZ wird die Pipettenspitze mehrfach in einer Waschlösung gespült, um ggf. vorhandene Reste vorheriger Lösungen zu entfernen.

### Variante A:

An dem Gerät befindet sich eine fest installierte Aufnahme Spitze für das "Andocken" (Aufsetzen) einer Pipettenspitze für die Pipettierung. Diese Pipettenspitze wird manuell vor dem Start des "Toponome Mapping System" bzw. "Toponome Mapping Verfahrens" (TMS) auf die fest installierte Aufnahmespitze aufgesetzt und anschließend wieder manuell entnommen. Zwischendurch wird kein Pipetten-Spitzen-Wechsel durchgeführt. Die Pipettenspitze wird je Einzelzyklus oder auch erst nach mehreren Zyklen des TMS Verfahrens von dem Geräte in Puffer gespült, um dann die nächsten Schritte des TMS Verfahrens durchzuführen.

### Variante B:

An dem Gerät befindet sich eine fest installierte Aufnahme Spitze für das "Andocken" (Aufsetzen) einer Pipettenspitze für die Pipettierung. Diese Pipettenspitze wird automatisch vor dem Start des TMS von dem Pipettiergerät aus einem Container aufgenommen, dann für den gesamten TMS Prozess verwendet und anschließend wieder automatisch, d.h. ohne manuelle Hilfe, wieder abgesetzt. Zwischendurch wird kein Pipetten-Spitzen-Wechsel durchgeführt. Die Pipettenspitze wird je Einzelzyklus oder auch erst nach mehreren Zyklen von dem Gerät in Puffer gespült, um dann die nächsten Schritte des TMS-Verfahrens durchzuführen.

### Variante C:

An dem TMS Gerät befindet sich ein fest installiertes Pipettiergerät, das eine fest installierte Pipettenspitze enthält, welche die verschiedenen Flüssigkeiten automatisch pipettiert. Zwischendurch wird kein Pipetten-Spitzen-Wechsel durchgeführt. Spülungen der Pipettenspitze erfolgen je nach Ablaufprotokoll während und/oder vor bzw. nach den Zyklen des TMS Prozesses.

### Variante D "Pipettiermodus Shear Flow":

Alle unter A bis C geschilderten Varianten können im Unterschied zu dem Verfahren in EP 0810 428 B1 im sog. Shear Flow Pipettiermodus eingesetzt werden. Dieser besteht darin, dass ohne jeden Pipettenspitzenwechsel mit ein und derselben Pipettenspitze die Inkubation eines jeden einzelnen Tags (Antikörper, Aptamer, Lectin etc.) rasch wiederholt wird, und zwar in der folgenden Weise:

Die von der Pipettenspitze aufgenommene Inkubationsflüssigkeit, die ein bestimmtes Tag enthält, wird auf die biologische Probe (auf dem Inkubationstisch des TMS) schräg aufgespritzt. Es entsteht auf der Probe ein Shear Flow der entsprechenden Inkubationslösung. Dieser führt zu einer verbesserten Tag Reaktion mit dem zu markierenden korrespondierenden Molekül in der Probe (Protein etc). Nach einer variabel programmierbaren, dann folgenden Inkubationszeit (z.B. 1 min) wird dieselbe Inkubationslösung rasch von derselben Pipettenspitze wieder abgesaugt, und anschließend in der beschriebenen schrägen Weise wieder auf dieselbe Probe gespritzt, usw. Dadurch entstehen N Zyklen der Inkubation mit ein und demselben Tag. Nach einer variabel zu bestimmenden Zeit wird dann mit einer Waschlösung gespült, gefolgt von dem Imaging-Vorgang, der die Lokalisation der Tag Reaktion in der Probe registriert.

Vorteil: Im Unterschied zu der klassischen "ruhenden" Dauerinkubation im Tropfen gemäß EP 0810 428 B1 wird die Effizienz der Markierungskinetik gesteigert, so dass der gesamte Inkubationsvorgang zeitlich stark verkürzt wird.

### Variation E:

Alle Verfahren 1A bis 1D werden entweder direkt auf dem Objekttisch im Strahlengang des Mikroskops oder extern (d.h. außerhalb des Strahlengangs des Mikroskops) durchgeführt (entweder auf dem Objekttisch des Mikroskops oder einem gesonderten Inkubationstisch des TMS Gerätes). Im Falle dieser sog. externen Inkubationsprozesse erfolgen die Imaging-Vorgänge immer erst nach positionsgenauer Rückführung des bzw. der zu untersuchenden Gesichtsfelder in den Strahlengang des Mikroskops. Im Falle der Verwendung von Fluorochromen, die an die jeweiligen Tags gekoppelt sind, nun im Falle der Verwendung von Bleichungsvorgängen, erfolgen die Bleichungsvorgänge entweder direkt im Strahlengang des Mikroskops oder extern in einer speziell installierten Bleichungsstation.

Alle Verfahrensvariationen 1A bis 1E können in Kombination mit den anderen Verfahren, die in den Punkten 3 bis 7 (siehe unten) beschrieben werden, erfolgen.

2. Automatisiertes Verfahren gemäß EP 0 810 428 B1 zur Durchführung der oben als Stand der Technik angegebenen repetitiven Inkubations-Imaging-Bleaching Zyklen (RIIBZ), mit dem einzigen Unterschied, dass gar kein Pipettierroboter, sondern ein kontinuierliches Schlauchsystem benutzt wird, um die RIIBZ durchzuführen. Dabei hat das Schlauchsystem folgende Merkmale:
a) Die zu messende(n) biologische(n) Probe(n) befindet(n) sich in einem gekammerten Bereich, der an ein zuführendes und ein abführendes Schlauchsystem angeschlossen ist.
b) Das Schlauchsystem ist an der zuführenden Seite an ein Druck-Pump-Gerät angeschlossen, das der Reihe nach Lösungen und Reagenzien in der in EP 0 810 428 B1 angegebenen Reihenfolge auf die gekammerte biologische Probe leitet. Ein jeweiliges Reagenz wird durch Druck über das zuführende Schlauchsystem auf die Probe gepumpt. Wenn das so zugeführte Reagenz die gekammerte Probe erreicht hat, wird der Pumpvorgang beendet, solange bis die Reaktion des Reagenz mit der gekammerten Probe abgeschlossen ist. Danach pumpt die Druckpumpe eine nächste Lösung (Waschlösung oder Reagenz) auf die Probe, dabei wird die vorherige Lösung in das abführende Schlauchsystem abgepumpt. Alternativ kann diese letztere Lösung durch eine Saugeinrichtung auf der abführenden Schlauchseite abgesaugt werden. Die abgeführte Flüssigkeit wird in einem gesonderten Gefäß aufgefangen. Auf diese Weise können beliebig viele Lösungen auf die Probe aufgebracht werden.

3. Semiautomatisiertes Verfahren, in dem alle Schritte der Datenerhebung wie in EP 0 810 428 B1 automatisiert durchgeführt werden, aber mir dem entscheidenden Unterschied, dass die einzelnen Bilder der einzelnen Markierungsschritte aus den Markierungszyklen nicht in derselben Vorrichtung überlagert werden, sondern manuell in ein zweites Gerät überführt werden. In diesem zweiten Gerät erfolgt dann die semiautomatische Überlagerung der Bilder.

4. Automatisiertes Verfahren gemäß EP 0 810 428 B1, jedoch mit dem entscheidenden Unterschied, dass keine Bleichungszyklen mehr erfolgen.

### Verfahren:

a) Alle Schritte werden so durchgeführt, wie in EP 0 810 428 B1 beschrieben. Es werden auch Fluoreszenzmarkierte Tags (Moleküle, die eine oder mehrere Bindungsstellen in der biologischen Probe erkennen) verwendet. Jedoch:
b) Nachdem ein erstes Bild von einem ersten Fluoreszenzverteilungsmuster eines ersten Markers registriert wurde, wird kein Bleichungsvorgang durchgeführt, sondern es werden sofort ein oder mehrere Waschschritte durchgeführt, und es wird dann sofort ein zweites Fluoreszenzmarkiertes Tag zu Inkubation gebracht. Das dann registrierte Bild enthält sowohl das nicht gebleichte Fluoreszenzsignal des ersten als auch des zweiten Fluoreszenz Tags. Auf diese Weise entsteht ein neues additives Fluozeszenzbild. Dieser Vorgang wird für n weitere Tags durchgeführt, so dass n Additivbilder entstehen. Der zunehmenden Fluoreszenzintensität wird durch das Signalaufnehmende System (CCD-Kamera o.ä.) dadurch entsprochen, indem der Dynamikbereich dieses Systems an die steigende Fluoreszenzintensität angepasst wird.

Um die spezifischen Markierungsmuster der einzelnen Tags aus den Additivbildern zu ermitteln, wird immer das einem einzelnen Tag-Bild jeweils vorausgehende Additivbild von dem jeweils letzten Additivbild subtrahiert. Dadurch bleibt das jeweils spezifische Tag-Bild "stehen". Es entspricht genau demjenigen Signal, das auch durch eine selektive Bildgebung nach einem jeweils einzelnen durchgeführten Bleichungsvorgang resultieren würde. Vorteil gegenüber EP 0 810 428 B1: Der gesamte Vorgang wird erheblich beschleunigt, da der Bleichungsvorgang wegfällt.

5. Automatisiertes Verfahren gemäß EP 0 810 428 B1, jedoch mit dem entscheidenden Unterschied, dass weder fluoreszierende Stoffe noch Bleichungszyklen Verwendung finden.

### Verfahren:

a) Es werden nur noch Inkubations-Imaging Zyklen durchgeführt gemäß EP 0 810 428 B1, jedoch werden nur Tags ohne Konjugation an irgendwelche optischen Signalgeber verwendet ("Label free" MELK).
b) In einem ersten Schritt wird ein Grauwertdurchlicht-Bild (Phasenkontrast und/ oder Interferenzkontrast) der Probe registriert. Danach wird dasselbe Objekt mit einer Fluoreszenzquelle angeregt, und das "unspezifische", in der Regel sehr schwache Fluoreszenzhintergrundbild wird registriert. Danach wird die Probe mit einem ersten "label free" Tag inkubiert. Es wird dann mit Waschlösungen gespült, und es wird ein zweites Durchlichtgrauwertbild erstellt, sowie danach ein entsprechendes Fluoreszenzanregungsbild von demselben Objekt registriert. Da das in der Probe gebundene Tag sowohl eine leichte Dichteänderung in der Probe erzeugt hat, als auch zu einem etwas anderen Fluoreszenzreflektionsverhalten geführt hat, entstehen zwei Bilddatensätze, die sich von den ersten beiden Bilddatensätzen unterscheiden. Durch Verrechnen dieser vier Datensätze, werden die Orte der spezifischen Tag-Bindung des ersten Tags in der Probe ermittelt. Es erfolgt dann die zweite Inkubation mit einem zweiten Label free Tag, usw..

Vorteil gegenüber EP 0 810 428 B1: Der gesamte Vorgang wird erheblich beschleunigt, da mehrere Arbeitsschritte wegfallen. Er wird außerdem kostengünstiger, da Tags nicht mehr konjugiert werden müssen.

6. Automatisiertes Verfahren gemäß EP 0 810 428 B1, jedoch mit dem entscheidenden Unterschied, dass keine Bleichungszyklen mehr durchgeführt werden, sondern dass die an die einzelnen Tags konjugierten fluoreszierenden Farbstoffe, die sich nach Inkubation in der Probe in Bindung befinden, mit Hilfe eines chemischen Agens aus dieser Bindung entfernt und dann weggewaschen werden, so dass keine Fluoreszenz mehr übrig bleibt.

### Verfahren

a) Es wird ein erstes fluoreszenzmarkiertes Tag inkubiert. Nach einem oder mehreren Waschschritten wird eine Lösung aufgebracht, die ein Agens (z.B. Enzym) enthält, welches die Bindungsstelle des Fluoreszenzmoleküls an das Tag exakt spaltet, so dass das Fluoreszenzmolekül in Lösung geht, während das Tag in Bindung verbleibt.
b) Nach (a) folgen ein oder mehrere Waschschritte, um das in Lösung gegangene Fluoreszenzmolekül zu entfernen. Es erfolgt dann die Inkubation mit dem nächsten fluoreszenzmarkierten Tag usw. Vorteil: der Bleichungsvorgang fällt weg.

7. Automatisiertes Verfahren zur Detektion des spezifischen Grauwertbereichs und der spezifischen Markierungsmuster einer einzelnen oder mehrerer Molekülspezies durch objektive Bestimmung des Bildhintergrunds.

Dieses Verfahren trägt der Tatsache Rechnung, dass jedwedes Tag in der Regel nicht nur aus spezifischen Bindungsstellen für ein bestimmtes Molekül in der zu markierenden biologischen Probe besteht, sondern neben einer oder mehreren solchen "gewünschten" Bindungs- "Domänen" auch noch andere, in der Regel schwächere Bindungsstellen enthält, die an andere als die gewünschten in der biologischen Probe binden. Diese letzteren Bindungseigenschaften beinhalten in der Regel sehr schwache Bindungskräfte, die meistens mehrere Größenordnungen kleiner sind als die "gewünschten". Dennoch stören diese Bindungsstellen das spezifische Bindungsverhalten der jeweiligen Tags dadurch, dass neben den stärkeren gewünschten auch die schwächeren nicht gewünschten schwachen Bindungen bei der Inkubation eintreten, so dass ein "Hintergrund-Rauschen" entsteht, das es quasi unmöglich macht, die spezifischen Bindungsstellen mit geringen Grauwerten (Stellen mit wenigen spezifischen Bindungsstellen in der Probe) von denjenigen geringen Grauwerten der schwachen ungewünschten Bindungen zu unterscheiden. Während also die starken Grauwertsignale keine Probleme der Entdeckung der gewünschten spezifischen Bindungen machen, ist eine sichere Trennung der spezifischen Bindungen von den unspezifischen (ungewünschten) im schwachen Grauwertbereich bisher nicht sicher möglich.

Durch folgendes Verfahren können diese Unterscheidungen erstmals getroffen werden:
a) Es wird ein fluoreszenzmarkiertes erstes ("blindes") Tag auf der biologischen Probe inkubiert, das dadurch gekennzeichnet ist, dass die spezifischen Bindungsdomänen fehlen, so dass es nur noch die anderen unspezifischen schwachen Bindungsdomänen enthält (z.B. nicht-immunes Antikörpermolekül, in dem die hypervariablen, also spezifisch bindenden Domänen entweder ganz fehlen, oder in der biologischen Probe nichts erkennen). Das nach einer bestimmten Inkubationszeit resultierende Fluoreszenzsignal wird registriert. Dann wird einmal oder mehrfach gespült, und das Signal wird solange gebleicht, bis kein weiteres Abfallen des Signals mehr registriert werden kann. Dann wird derselbe Vorgang mit demselben Tag in derselben Konzentration wiederholt. Es wird nach Spülvorgängen wiederum das resultierende Bild registriert. Dieser Vorgang wird solange wiederholt bis kein weiterer Signalanstieg mehr registriert wird. Dann wird dieses letzte Signal solange gebleicht bis keine weitere Signalreduktion mehr auftritt. Erst danach wird das nächste Tag derselben Molekülklasse, allerdings diesmal mit der voll bindungsfähigen spezifischen Bindungsstelle inkubiert. Als Resultat ergibt sich dann ein Summenbild, das einerseits aus Grauwertsignalen sowohl der ungewünschten unspezifischen Bindungsstellen (soweit diese nach den vorausgegangenen Zyklen nach Absättigung überhaupt noch in der Probe vorhanden sind) als auch der spezifischen Signalmuster besteht. Dann erfolgt eine Fortsetzung weiterer Markierungen durch Tags derselben Molekülklasse gemäß EP 0 810 428 B1. Das Verfahren kann beliebig dadurch modifiziert werden, indem zu jedem beliebigen Zeitpunkt während dieses Gesamtvorgangs wiederum ein "blindes" Tag inkubiert werden kann, um das Hintergrundbindungsverhalten zu überprüfen. Auf diese Weise sind zahlreiche, an den jeweiligen biologischen Fragestellungen orientierten Variationen dieses Verfahrens möglich. Eine besondere Variation besteht darin, dass die "blinden" Tags nicht vor den Zyklen der spezifischen Tags, sondern erst nach der Beendigung dieser "spezifischen" Zyklen angeschlossen werden. Durch Vergleich von Zyklen mit vorausgeschalteten "blinden" Tags mit Zyklen, in denen die "blinden" Tags nachgeschaltet werden, kann ermittelt werden, ob die vorausgehenden Bindungsstellen der "blinden" Tags ggf., die spezifischen Bindungsstellen der spezifischen Tags teilweise oder vollständig maskieren.
b) Das spezifische Bindungsmuster für jedwedes Tag derselben Molekülklasse kann dann dadurch ermittelt werden, indem die Grauwertbilder der unspezifischen "blinden" Tags mit den Grauwertbildern der spezifischen Tags verrechnet werden, so dass nur die eindeutig spezifischen Bindungsmuster übrig bleiben. Andererseits können auch Grauwertbereiche der spezifischen Tags, die mit den "blinden" Tags überlagern, im Wege der Bildverarbeitung von dem visualisierten Grauwertbereich der spezifischen Tags ausgeschlossen werden.

Vorteil: Es können auf diese Weise sehr genaue Aussagen über das spezifische Bindungsverhalten eines jedweden Tags über einen weiten Grauwertbereich getroffen werden, so dass es erstmals ohne weiteres möglich ist, spezifische Schwellen-Grauwerte für jedes einzelne Tag zu ermitteln, oder sogar den gesamten Grauwertbereich jedes Tags als multidimensionalen Vektor zu ermitteln, um durch Datenpunkt-genaue Überlagerung dann die kombinatorischen multidimensionalen Vektoren aller spezifischen Bindungsstellen zu ermitteln. Es entsteht ein quantitatives Abbild der tatsächlichen relativen Konzentrationsbeziehungen der gemessenen verschiedenen Moleküle zueinander in jedem einzelnen Datenpunkt der biologischen Probe, als eine genaue Abbildung der realen biologischen Situation.

### Schlussfolgerung

Die dargelegten Verfahren können einzeln oder kombiniert als Gesamtprozess eingesetzt werden, der hier als "Toponome Mapping System" (TMS) bezeichnet wird. Dadurch erfährt der bisherige Stand der Technik, repräsentiert durch die MELK Technologie (EP) eine entscheidende Erweiterung und Verbesserung in folgender Hinsicht:
1. Effizienzsteigerung: es können bis zu 10 Proteine pro Stunde gemessen werden. Das erhöht die Effizienz des bisherigen MELK Verfahrens um Faktor 5 (von 2 Proteinen pro Stunde auf ca. 10 Proteine pro Stunde); 2. Modularität: Die verschiedenen Verfahrensteile können beliebig kombiniert werden: Dadurch wird das TMS sehr anpassungsfähig an die jeweilige biologische Fragestellung oder die Art der zu lokalisierenden Proteine oder anderen Gewebe- und Zellbestandteile. 3. Objektive molekulare Kartierung: Ein solches TMS erhält durch die Möglichkeit, innerhalb des Meßprozesses biologisch-molekulare Parameter des objektiven Hintergrunds "einzubauen" (molekular-basierte Bild-Hintergrund-Dissektion) die Fähigkeit, Grauwertbereiche für jedes einzelne Protein pro Meßdatenpunkt (Pixel oder Voxel) genau zu charakterisieren, d.h. es können - quasi aufgesetzt auf derartige TMS Datensätze - andere Verfahren der mathematischen/informationstechnischen Analyse (z.B. Verfahren der Kombinatorik und Geometrie) eingesetzt werden, die dann sicher sein können, nicht im Rauschen zu analysieren. Das verbessert die Auswertung von TMS Datensätzen 4. Einsatzfähigkeit für die klinische Routine: das Verfahren erhält durch die automatisierbare Auswertung von Proteinsignalen (objektive Trennung von Vordergrund und Hintergrund) zusammen mit der Durchsatzsteigerung um mindestens Faktor 5 die für einen klinischen Einsatz, z.B. in der Diagnostik, erforderliche Robustheit, Reproduzierbarkeit, und Geschwindigkeit.

## Patentansprüche

1. Verfahren zur Bestimmung und Messung einer beliebigen Anzahl Xn (n = 1,2,3...N) von Zielstrukturen bestehend aus Molekülklassen, Molekülgruppen und Molekülteilen auf einem flüssigen oder festen Objekt bzw. in einem flüssigen oder festen Objekt mittels repetitiver Inkubations-Imaging-Zyklen, **dadurch gekennzeichnet, dass** das Verfahren folgende Schritte umfasst:
a) Aufbringen eines Fluoreszenzmarkierten Tags auf das Objekt und Ermittelung des resultierenden Fluoreszenzmarkierungsmusters;
b) Aufbringen von mindestens einer Waschlösung auf die Probe;
c) Aufbringen von mindestens einem chemischen Agens auf das Objekt, wobei das Agens die Bindungsstelle das Fluoreszenzmoleküls an das Tag spaltet;
d) Wiederholen der Verfahrensschritte a) bis c) für beliebig viele markierte Tags.
